# EUROPEAN PATENT APPLICATION

(11) **EP 1 970 089 A2**
(43) Date of publication of application: **17.09.2008**
(21) Application number: 07150026.8
(22) Date of filing: 22.08.2003
(51) Int. Cl.: A61M 25/01, A61M 25/00

(54) **Coronary vein navigator**

(30) Priority: 23.08.2002 US 226647
(62) Divisional of application: 03793298.5
(71) Applicant: Cardiac Pacemakers, Inc., St. Paul MN 55112 (US)
(72) Inventor: Tockman, Bruce, Scandia, WI 55073 (US); Hall, Jeffrey A., Birmingham, AL 35244 (US); Westlund, Randy, River Falls, WI 54022 (US)
(74) Representative: Peterreins, Frank

(57) **Abstract**

A guide catheter system comprises a guide catheter comprising a flexible shaft defining a longitudinal axis and having a proximal end, a distal end, and a main lumen; a navigator catheter having a proximal end, a distal end, and a central lumen, the navigator catheter longitudinally displaceable within the main lumen of the guide catheter, the distal end of the navigator catheter dimensioned for passage into an angled vein distal to a patient's coronary sinus ostium, the central lumen dimensioned to receive a longitudinally displaceable guide wire; and a deflection arrangement provided at the distal end of the navigator catheter for directing the guide wire into the angled vein, the deflection arrangement imparting a bend at the distal end of the navigator catheter having an angle sufficient to facilitate passage of the distal end of the navigator catheter into the angled vein, wherein the deflection arrangement comprises a deflection mechanism that imparts the bend angle, the deflection mechanism controllable from the proximal end of the navigator catheter.

## Description

### FIELD OF THE INVENTION

The invention relates generally to guide catheters, and, more particularly, to a coronary vein navigator catheter apparatus for accessing coronary vessels distal of the coronary sinus ostium.

### BACKGROUND OF THE INVENTION

Guiding catheters are instruments that allow a physician to locate and cannulate vessels in a patient's heart for performing various medical procedures, including venography and implanting of cardiac leads. Cannulating heart vessels requires navigating a small diameter, flexible guide through convoluted vasculature to access a destination heart vessel. Once the destination heart vessel is reached, the catheter acts as a conduit for insertion of payloads into the vessel.

A commonly accessed destination vessel for cardiac pacing lead insertion is the coronary sinus. A number of guiding catheter implementations have been developed for locating and accessing the ostium of the coronary sinus. In addition to the difficulties associated with accessing the coronary sinus, certain cardiac management devices, such as resynchronizers for example, require that the physician navigate a guiding catheter beyond the coronary sinus and into a coronary vein, such as the great cardiac vein, to facilitate lead implantation on the left ventricle. Guiding catheters that are well suited for accessing the coronary sinus may not be suitable for left-side coronary vein navigation.

By way of example, lateral and posterior branches of the coronary sinus and great cardiac vein often branch at acute, right or obtuse angles from a main vessel. To access such highly angled vessels, a guide wire is often used. However, the diameter of the main vessel can be very large in heart failure patients, for example. As such, the main vessel provides no back support for a guide wire to push off from when attempting to turn the guide wire into a side branch.

There is a need for an improved catheter apparatus and method of using same that can be used to efficiently navigate coronary vessels, particularly left-side coronary vessels. The present invention fulfills these and other needs, and addresses other deficiencies of prior art implementations and techniques.

### SUMMARY OF THE INVENTION

The present invention is directed to a system and method for navigating a catheter apparatus through coronary vasculature. According to one embodiment, a guide catheter system includes a guide catheter having a flexible shaft defining a longitudinal axis, a proximal end, a distal end, and a main lumen. The guide catheter system further includes a navigator catheter having a proximal end, a distal end, and a central lumen. The navigator catheter is longitudinally displaceable within the main lumen of the guide catheter.

The distal end of the navigator catheter is dimensioned for passage into an angled vein distal to a patient's coronary sinus ostium, and the central lumen is dimensioned to receive a longitudinally displaceable guide wire. A deflection arrangement is provided at the distal end of the navigator catheter for directing the guide wire into the angled vein. The deflection arrangement, which can be static or controllable, imparts a bend at the distal end of the navigator catheter having an angle sufficient to facilitate passage of the distal end of the navigator catheter into the angled vein. The bend angle can be an acute angle, a 90 degree angle or an obtuse angle relative to a longitudinal axis of the navigator catheter proximal of the deflection arrangement.

According to another embodiment of the present invention, a guide catheter system includes a guide catheter having a flexible shaft defining a longitudinal axis, a proximal end, a distal end, and a main lumen. A navigator member includes a proximal end and a distal end. The navigator member is longitudinally displaceable within the main lumen of the guide catheter, and the distal end of the navigator member is dimensioned for passage into an angled vein distal to a patient's coronary sinus ostium. A deflection arrangement is provided at the distal end of the navigator member. The deflection arrangement imparts a bend at the distal end of the navigator member having an angle sufficient to facilitate passage of the distal end of the navigator member into the angled vein.

In accordance with a further embodiment, a guide catheter system includes a guide catheter having a flexible shaft, a proximal end, a distal end, and a main lumen. A navigator catheter includes an outer wall having an aperture, a central lumen, a proximal end, and a distal end. The navigator catheter is longitudinally displaceable within the main lumen of the guide catheter. The distal end of the navigator catheter is dimensioned for passage into a cardiac vein distal to a patient's coronary sinus ostium. A deflection member is disposed within the central lumen of the navigator catheter proximate the aperture of the outer wall. The deflection member is oriented at an angle relative to a longitudinal axis of the navigator catheter sufficient to deflect a guide wire passed within the central lumen through the aperture of the outer wall of the navigator catheter and into an angled vein branching from the cardiac vein.

According to yet another embodiment of the present invention, a method of navigating coronary vasculature involves providing a guide catheter system which includes a guide catheter, a navigator catheter longitudinally displaceable within the guide catheter, and a deflection arrangement provided at a distal end of the navigator catheter. The method further involves advancing the guide catheter to at least a patient's coronary sinus ostium, and extending the navigator catheter from the guide catheter to a location proximate or within an angled vein distal to the coronary sinus ostium. Using the deflection arrangement, a guide wire passing through the navigation catheter is directed into the angled vein. A lead having an open lumen is advanced over the guide wire to direct the lead to an implant site within the angled vein.

In accordance with a further embodiment, a method of navigating coronary vasculature involves providing a guide catheter system which includes a guide catheter, a navigator catheter longitudinally displaceable within the guide catheter, and a deflection arrangement provided at a distal end of the navigator catheter. The method further involves advancing the guide catheter to at least a patient's coronary sinus ostium, and extending the navigator catheter from the guide catheter to a location proximate an angled vein distal to the coronary sinus ostium. The navigator catheter is seated within the angled vein. The guide catheter is passed over the navigator catheter to advance the guide catheter into the angled vein. The navigator catheter is retracted from the guide catheter, and a lead is advanced through the guide catheter to an implant site within the angled vein.

The above summary of the present invention is not intended to describe each embodiment or every implementation of the present invention. Advantages and attainments, together with a more complete understanding of the invention, will become apparent and appreciated by referring to the following detailed description and claims taken in conjunction with the accompanying drawings.

### BRIEF DESCRIPTION OF THE DRAWINGS

Figure 1 is a cut-away view of a patient's heart, showing a guide catheter apparatus embodying features of the present invention deployed within the heart;
Figures 2A-2C illustrate embodiments of a guide catheter apparatus employing a navigator catheter having a pre-formed distal end;
Figures 3A and 3B illustrate embodiments of a guide catheter apparatus employing a navigator catheter having a flexible, formable distal end;
Figure 4 illustrates an embodiment of a guide catheter apparatus employing a guide catheter and a navigator catheter each having a pre-formed distal end;
Figures 5A and 5B illustrate an embodiment of a guide catheter apparatus employing a navigator catheter having a steering or pulling arrangement for controllably changing a bend angle or shape of a distal region of the navigator catheter;
Figures 6A and 6B illustrate an embodiment of a guide catheter apparatus employing a navigator catheter having an inflation mechanism for controllably changing a bend angle or shape of a distal region of the navigator catheter;
Figure 7 illustrates an embodiment of a guide catheter apparatus employing a navigator catheter having a deflection member for redirecting a guide wire through an exit aperture at a prescribed exit angle;
Figures 7B-11B illustrate an embodiment of a guide catheter apparatus employing a navigator catheter having a controllable deflection member for redirecting a guide wire through an exit aperture at a multiplicity of selectable exit angles; and
Figures 12-14 illustrate an embodiment of a guide catheter apparatus employing a guide catheter and a navigator catheter that cooperate to access a left-side coronary vessel in accordance with the present invention.

While the invention is amenable to various modifications and alternative forms, specifics thereof have been shown by way of example in the drawings and will be described in detail herein. It is to be understood, however, that the intention is not to limit the invention to the particular embodiments described. On the contrary, the invention is intended to cover all modifications, equivalents, and alternatives falling within the scope of the invention as defined by the appended claims.

### DETAILED DESCRIPTION OF THE VARIOUS EMBODIMENTS

In the following description of the illustrated embodiments, references are made to the accompanying drawings which form a part hereof, and in which is shown by way of illustration, various embodiments in which the invention may be practiced. It is to be understood that other embodiments may be utilized, and structural and functional changes may be made without departing from the scope of the present invention.

A coronary vein guide catheter system of the present invention employs a navigator catheter or member in combination with a guide catheter to effectively navigate coronary vasculature having sharply angled vessels. As was discussed previously, it is often necessary to direct a guide wire to make a 90 degree or other sharp angled turn when attempting to reach a desired implant site, such as on the left ventricle. Traditional techniques for effecting sharp turns with a guide wire require close proximity between the guide wire and a vessel wall. Such techniques require contact between the guide wire and vessel wall to re-direct the guide wire in a direction needed to access a branch vessel.

In many circumstances, however, the primary vein from which the vein of interest branches is relatively large in comparison to the branch vein. For example, a sharply angled vein of interest may branch off of the coronary sinus or great cardiac vein. Because the diameter of the coronary sinus or great cardiac vein is many times larger than the diameter of the guide wire, the wall of the coronary sinus or great cardiac vein cannot effectively be used to assist in steering the guide wire into the branch vein. In such cases, a significant amount of time and skill is required on the part of the physician to successfully access such as a branch vein.

In accordance with one approach, a guide catheter system of the present invention employs a navigator catheter to advantageously direct a guide wire into a sharply angled branch vessel irrespective of the size of the primary vessel leading to the vessel vein. As such, the physician need not possess specialized navigation skills to efficiently navigate tortuous cardiac vasculature, such as left-side blood vessels. Employing a guide catheter system of the present invention provides for quicker navigation of difficult venous anatomy by the average skilled physician.

By way of example, and in accordance with one technique of the present invention, the guide catheter system is introduced into a patient's heart and advanced to pass into or through the coronary sinus. The navigator catheter or member is extended from the guide catheter and is positioned at a take off of a branch vein or is inserted into the take off of a branch vein distal to the coronary sinus ostium. A relatively small diameter guide wire (e.g., ≤ 0.018 inches) is then advanced into the branch vein through the navigator catheter, and the navigator catheter is then retracted. A coronary venous lead is then inserted over the proximal end of the guide wire and advanced to the target implant site. After lead implantation, the guide wire and guide catheter are retracted.

According to another technique of the present invention, a navigator catheter or member and guide catheter cooperate to access left-side coronary vasculature for implanting a lead in a manner which obviates the need for an over-the-wire lead implant technique. A navigator catheter or member is extended from the guide catheter situated within or distal to the coronary sinus to a position proximate a take off of a branch vein. The navigator catheter, which may have an open lumen or a closed lumen at its distal end, or the navigator member is maneuvered around the bend angle of the branch vein and advanced into the branch vein. In the case of an open lumen configuration, a relatively large diameter guide wire (e.g., 0.030-0.038 inches) can be advanced through the open lumen of the navigator catheter to assist in accessing the branch vein of interest. However, according to this embodiment, the guide wire is retracted after the navigator catheter is advanced into the branch vein of interest and not used as part of the lead implant procedure.

After the navigator catheter or member is seated in the coronary vein of interest, the guide catheter is then advanced over the navigator catheter or member so that the guide catheter is advanced past the bend angle of the destination vein and into the destination vein. The navigator catheter or member is then retracted from the guide catheter and a medical electrical lead is advanced through the guide catheter to the implant site. The lead is then implanted, and the guide catheter removed. It is to be understood that, although features of the present invention will generally be described with reference to veins of the heart, that such features are also applicable in the context of arteries of the heart, as well as other vessels of the body.

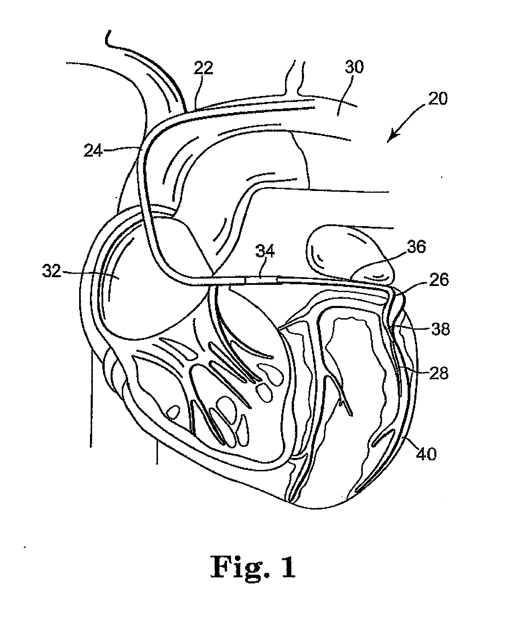

With reference to Figure 1, a guide catheter system employing a guide navigator catheter is illustrated in accordance with an embodiment of the present invention. The guide catheter system 22 includes a navigator catheter 26 and a guide catheter 24. The guide catheter system 22 is shown deployed within a patient's heart. As shown, the guide catheter system 22 is introduced into the patient's subclavian vein 30 and into the right atrium 32. The physician uses the guide catheter system 22 to access the coronary sinus 34 via the right atrium 32. A distal end of the guide catheter 24 and/or the navigator catheter 26 is used to locate and access the ostium of the coronary sinus 34.

Having accessed the coronary sinus 34, the navigator catheter 26 is advanced within the guide catheter 24 so that the distal end of the navigator catheter 26 extends beyond the distal end of the guide catheter 24. The navigator catheter 26 employs a deflection arrangement to access a cardiac vein distal from the coronary sinus ostium. For example, a pre-shaped or shape-controlled distal end of the navigator catheter 26 is maneuvered into a vein that branches at a sharp angle from the coronary sinus or other cardiac vein, such as the great cardiac vein. After the navigator catheter 26 has been advanced into the branch vein, a guide wire 28 can be advanced through the guide and navigation catheters 24, 26 to a site 40 appropriate for lead implantation on the left ventricle.

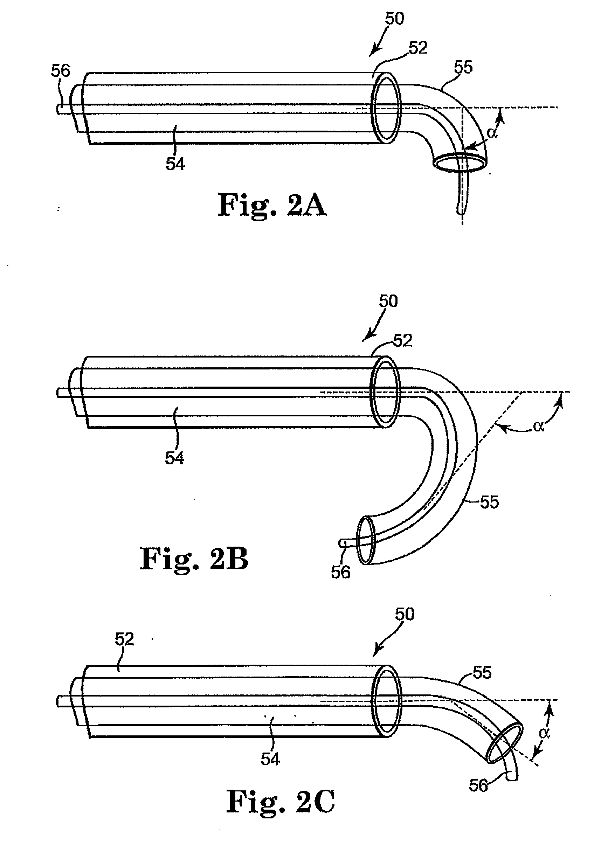

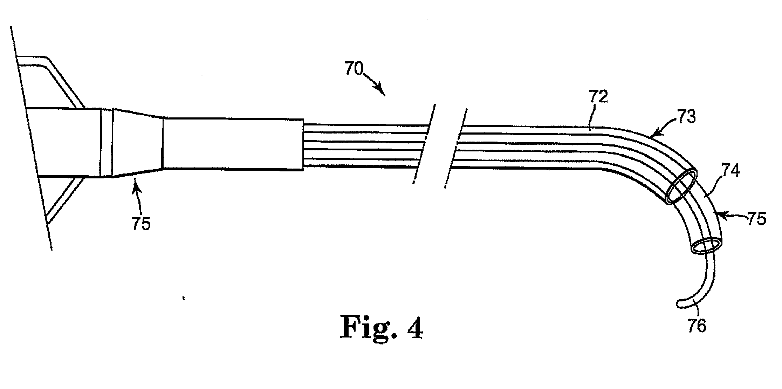

Referring now to Figure 2B, an embodiment of a guide catheter system is shown embodying features of the present invention. A navigator catheter 54 is movably disposed within an open lumen of a guide catheter 52, such that the navigator catheter 54 can translate longitudinally and, if desired, rotate axially within the guide catheter 52. The navigator catheter 54 may include a proximal attachment to facilitate manipulation of the navigator catheter 54. In the embodiment shown in Figure 4, for example, the proximal attachment includes a wing luer 75, although other suitable proximal mechanisms may be employed. In one configuration, the navigator catheter 54 includes an open lumen, and the open lumen can be adapted to receive a payload. In the context of a guide wire navigator embodiment, the open lumen of the navigator catheter 54 is dimensioned to receive a guide wire 56.

As will be described hereinbelow, in other applications in which the navigator catheter 54 is employed to access a sharply angled coronary branch vein without use of a guide wire, the lumen of the navigator catheter 54 can be closed at its distal end. According to further applications, a navigator member 54, such as a solid member as in the case of a stylet, is employed to facilitate access of sharply angled coronary branch veins, rather than use of a catheter. These and other implementations will be discussed hereinbelow.

The guide catheter 52 and navigator catheter 54 are configured with dimensions appropriate for the intended venous/arterial access path of a given medical procedure. For example, in the context of left-side cardiac access applications, the guide catheter 52 may be formed with an outer diameter from about 6 French to about 10 French, and have a length of about 40 cm to about 60 cm. The navigator catheter 54 may be formed with an outer diameter smaller than that of the guide catheter 52, and may range from about 3 French to about 8 French and have a length longer than that of the guide catheter. In one configuration particularly useful in accessing coronary veins distal to the coronary sinus ostium, the navigator catheter 54 can have an outer diameter of about 6 French and the guide catheter 52 can have an outer diameter of about 8 French. It is understood that these exemplary dimensions are provided for purposes of illustration only, and not of limitation.

The guide catheter 52 and navigator catheter 54 are typically formed of a molded elastomeric tubing. An appropriate elastomeric material, such as a high durometer Pebax, urethane or epoxy, can provide the desired longitudinal stiffness. It is also possible to include an inner lubricious lining, formed from a material such as PTFE, or a lubricious coating, such as a hydrophilic coating, on an inner surface of the catheter tubing. The guide catheter 52 and navigator catheter 54 may also include a soft distal tip to prevent tissue abrasion along the venous pathways.

In other implementations, the guide catheter 52 and navigator catheter 54 can be constructed according to a multi-layer tube design. For example, one particular multi-layer tube design includes an inner lubricious liner, a braid, and an outer jacket. The lubricious liner is typically formed from a material such as PTFE and is disposed within an open lumen of the catheter shaft. The braid is typically located between the lubricious liner and outer jacket. The braid can provide longitudinal stiffness and requisite torque transmission to facilitate rotation and longitudinal advancement of the catheters 52, 54 through blood vessels, as well as helping to prevent kinking of the catheter shafts. The braid is usually constructed from a weave of stainless steel wire or ribbon, although a non-metallic fiber braid can also be employed, such as a braid formed to include polymer fibers (e.g., KEVLAR). The outer jacket is typically a high durometer polymer such as Pebax, urethane or epoxy, as previously discussed. The outer jacket provides the catheters 52, 54 with a smooth and durable outer surface.

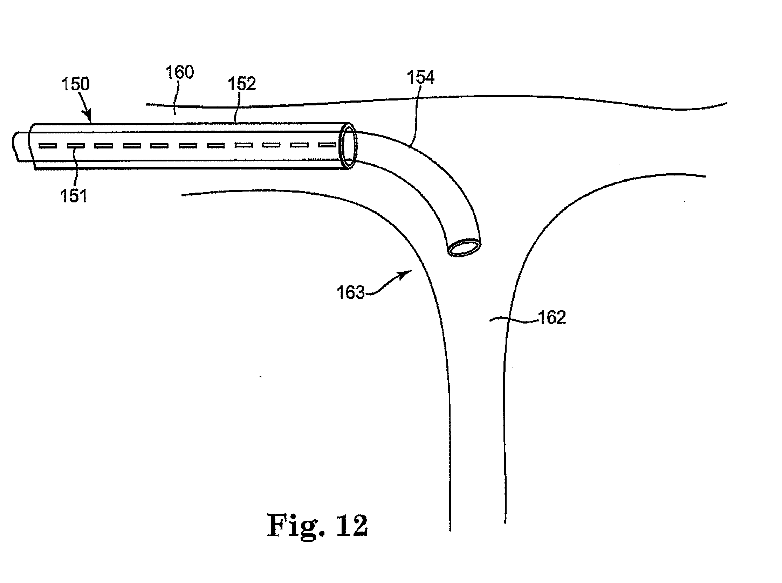

In certain configurations, the guide catheter 52 can include a longitudinal pre-stress line, such as pre-stress line 151 shown in Fig. 12, that extends between the distal and proximal ends of the guide catheter 52. The pre-stress line is typically a V-shaped notch or groove formed on a surface of the guide catheter 52. Other configurations of a pre-stress line are possible, such as a fiber or wire longitudinally embedded within the guide catheter 52. The pre-stress line provides for splitting of the guide catheter 52 to facilitate retraction of the guide catheter 52 from the patient. Two pre-stress lines can also be employed, the two pre-stress lines typically being distributed oppositely (180 degrees apart) around a transverse cross sectional perimeter of the guide catheter 52. Inclusion of one or more pre-stress lines provides for peel-away retraction of the guide catheter 52 after lead implantation.

The splitting of the guide catheter 52 is beneficial as it allows the guide catheter 52 to be removed without the disturbing any attachments that may be mounted on the proximal end of navigator catheter 54. For example, a wing luer 75 (best seen in Fig. 4), may be mounted to the proximal end of the navigator catheter 54. Splitting the guide catheter 52 during retraction enables the guide catheter 52 to be retracted without interfering with the wing luer 75.

Figures 2A-2B illustrate embodiments of a guide catheter system 50 which employ a navigator catheter 54 having a pre-formed shape 55 at a distal end of the navigator catheter 54. In general terms, the profile and dimensions of the pre-shaped distal bend 55 are particular to the intended guiding application. The pre-shaped distal bend 55 can be thermoset on the flexible navigator catheter 54 during manufacture.

The pre-formed portion 55 of the distal end of the navigator catheter 54 is more compliant that the guide catheter 52. As such, the pre-shaped distal bend 55 of the navigator catheter 54 tends to straighten when inserted into the guide catheter 52, which facilitates advancement of the navigator catheter 54 through the guide catheter 52. When the navigator catheter 54 is extended beyond the guide catheter 52, the navigator catheter's distal end takes on the shape of the pre-formed curve imparted thereat.

In applications involving left-side coronary veins distal to the coronary sinus ostium, for example, the bend angle, α, can be selected to gain access to particular branch veins having sharp access angles. Figures 2A-2C show three configurations of a navigator catheter 54 having different bend angles, α. Figure 2A depicts a navigator catheter 54 having a pre-formed distal bend 55 which forms an angle, α, of about 90 degrees relative to a longitudinal axis of the guide catheter 52 or the navigator catheter 54 proximal of the pre-formed distal bend 55. Figure 2B depicts a navigator catheter 54 having a pre-formed distal bend 55 which forms an obtuse angle, α, relative to the longitudinal axis of the guide catheter 52 or the navigator catheter 54 proximal of the pre-formed distal bend 55. Figure 2C depicts a navigator catheter 54 having a pre-formed distal bend 55 which forms an acute angle, α, relative to the longitudinal axis of the guide catheter 52 or the navigator catheter 54 proximal of the pre-formed distal bend 55. In most applications, the bend angle, α, imparted at the distal end of the navigator catheter 54 can range from about 0 degrees to about 180 degrees or more.

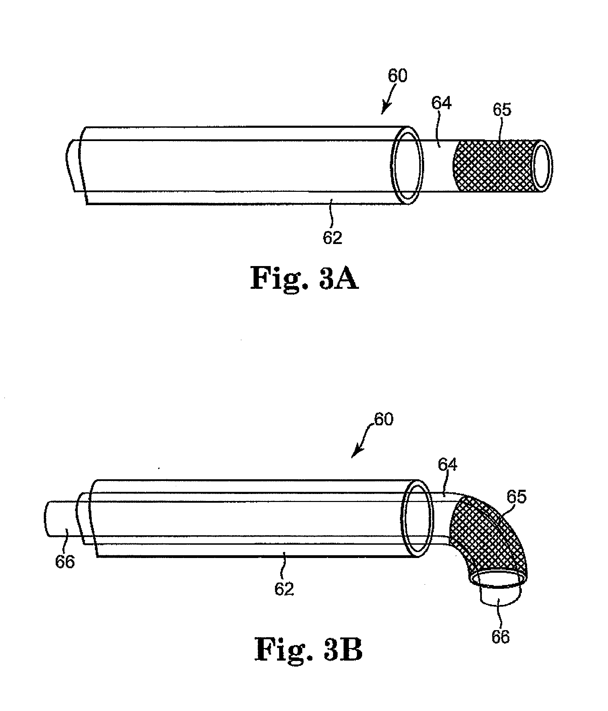

Figures 3A and 3B illustrate a coronary vein guide catheter system 60 according to another embodiment of the present invention. According to this embodiment, a navigator catheter 64 of the guide catheter system 60 includes a flexible distal end 65. In this configuration, the distal end 65 does not include a pre-formed distal bend, as in the embodiments in Figs. 2A-2C. Rather, the flexible distal end region 65 is sufficiently flexible to assume the shape of the distal portion of a shaping member 66 when the shaping member 66 is advanced into and/or through the flexible distal end region 65.

In typical use, the navigator catheter 64 is extended beyond the distal end of the guide catheter 62 and toward a coronary branching vein of interest. A shaping member 66, such as a core guide wire or shaping wire, is advanced through the guide catheter 62 and navigator catheter 64, and into or past the flexible distal end 65. It is noted that the pre-formed distal end of the shaping member 66 can be more compliant than the guide catheter 62 and navigator catheter 64 to permit straightening thereto to facilitate advancement of the shaping member 66 though the catheters 62, 64. The shape imparted to the flexible distal end 65 of the navigator catheter 64 facilitates locating and accessing of the branch vein of interest.

After the flexible end 65 is advanced a sufficient distance into the branch vein, the shaping member 66 is retracted. It is understood that a guide wire may be used with the navigator catheter 64 of this embodiment to enhance locating and accessing of the coronary vein of interest. In addition, the guide wire may be employed to facilitate over-the-wire implanting of a medical electrical lead in the subject coronary vein. Alternatively, a larger diameter guide wire can be used solely for coronary vein access, and not during lead implantation.

One particular advantage of this configuration is the ability to develop a multiplicity of acute and obtuse bend angles at the distal end of the navigator catheter by selective employment of shaping members 66 having different bend angles. As such, only the shaping member 66 need be retracted and substituted to modify the bend angle of the navigator catheter's distal end, thereby obviating the need to remove and substitute the navigator catheter itself to achieve this objective.

Figure 4 illustrates an embodiment in which a navigator catheter 74 cooperates with a guide catheter 72 having a pre-formed distal end to enhance access to the coronary sinus and coronary veins distal to the coronary sinus ostium. A guide wire 76 may also be employed for catheter navigation and, if desired, lead implantation. In this embodiment, the distal end of the guide catheter 72 has a pre-shaped region 73 that can take on a variety of bend angles depending on a particular application.

The guide catheter system 70 is shown to include a guide catheter 72 having an open lumen and a pre-formed distal end 73. A navigator catheter 74 having an open lumen and a pre-formed (e.g., Figs. 2A-2C) or formable (e.g., Figs. 3A-3B) distal end 75 is movably disposed within the open lumen of the guide catheter 72. The shaped distal end 75 of the navigator catheter 74 is more flexible than the distal end 73 of the guide catheter 72. The guide catheter system 70 further includes a proximal mechanism 75 used for axially rotating the guide catheter 72 relative to the navigator catheter 74 and longitudinally translating the navigator catheter 74 relative to the guide catheter 72. The axial rotation and longitudinal translation allows the distal end section of the guide catheter system 70 to assume a selectable multiplicity of two- and three-dimensional shapes appropriate for accessing the coronary sinus and coronary vessel of interest distal to the coronary sinus ostium. Additional details concerning these and other enhancing features are described in commonly owned, co-pending applications identified under U.S. Serial Nos. 10/059,809 filed January 28, 2002, 10/105,087 filed March 22, 2002, and 10/011,084 filed December 6, 2001, each of which is hereby incorporated by reference herein in its respective entirety.

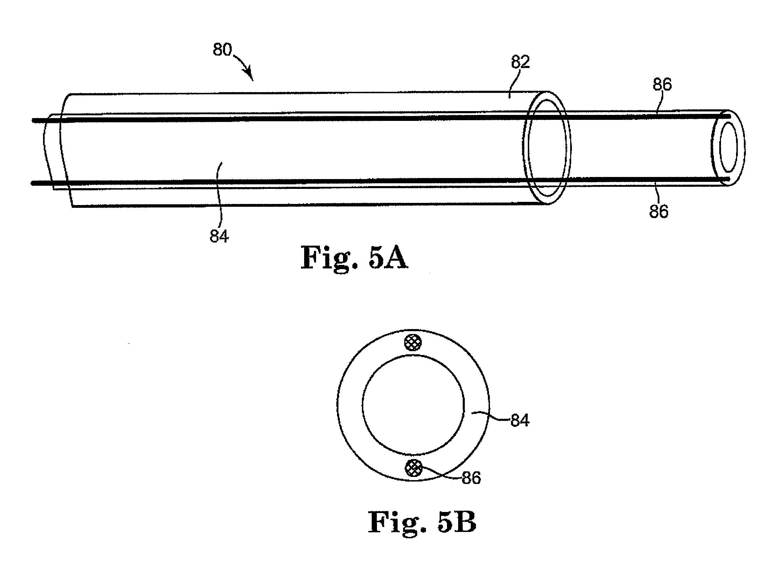

Turning now to Figs. 5A and 5B, there is shown an embodiment of a coronary vein guide catheter system 80 which includes a navigator catheter 84 having a deflection mechanism that provides for an adjustable bend angle and/or shape at the distal end of the navigator catheter 84. The deflection mechanism can be controlled by the physician to control the shape of the distal end of the navigator catheter 84. Bend angles of between 0 degrees and 180 degrees or more can be achieved to facilitate locating and navigation of cardiac structures and vessels of interest, such as the coronary sinus ostium and coronary vein and branch veins distal to the coronary sinus ostium.

According to one embodiment, the deflection mechanism of the guide catheter system 80 includes one or two steering tendons 86 that extend from the distal tip of the navigator catheter 84 and are accessible by the physician at the proximal end of the navigator catheter 84. The steering tendons 86 are typically situated within respective satellite lumens. In general, the shape of the distal end of the navigator catheter 84 can be altered by applying tension to one or both steering tendons 86. The navigator catheter 84 can be configured to be generally straight when no tension is applied to the tendons 86, but may alternatively be fabricated to include a pre-formed shape at its distal end.

When steered, the distal end of the navigator catheter 84 can assume a variety of simple and complex shapes, including, for example, a semicircular arc or even a full circular shape whose radius of curvature depends upon the amount of tension applied to the steering tendon 86. Employment of a shape altering deflection mechanism within the guide catheter system 80 provides for efficient coronary vein locating, accessing, and lead implantation.

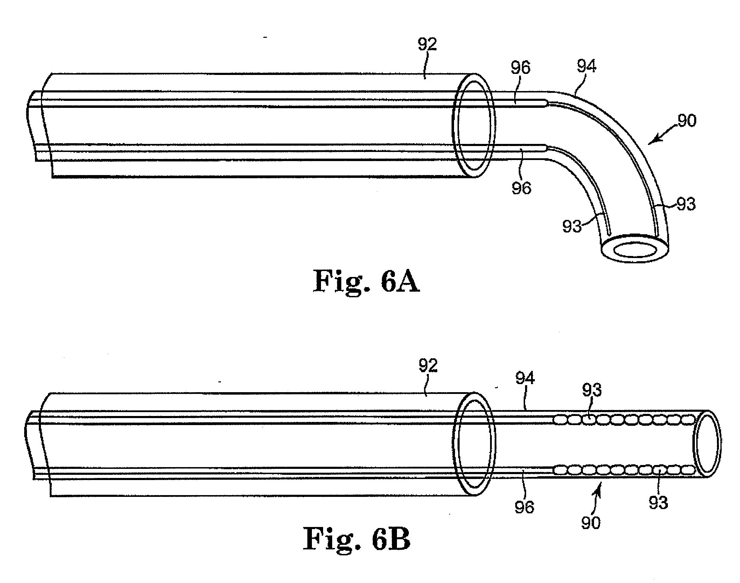

In accordance with another embodiment, and with reference to Figs. 6A and 6B, the deflection mechanism employed in the guide catheter system 90 can include a hydraulic mechanism that controls the bend angle/shape of the distal end of the navigator catheter 94. The navigation catheter 94 may be formed to include a pre-shaped distal bend. According to this embodiment, one or more inflation members 93 are situated at the distal end of the navigator catheter 94 to effect shape changes to the catheter's distal end. The inflation members 93 are in fluid communication with an inflation mechanism (not shown) situated at the proximal end of the navigator catheter 94 via inflation lumens 96. Multiple inflation members 93 may be employed to effect more complex shapes and bend angles at the distal end of the navigation catheter 94, in which case two or more inflation lumens 96 may be used.

The inflatable members 93 are in fluid connection with the inflation lumens 96. The inflatable members 93 change a shape of the pre-shaped distal bend of the navigator catheter 94 upon inflation and deflation. The inflatable members 93 can be arranged to encompass a partial circumferential angle of a cross section of the navigation catheter 94. The partial circumferential angle in this arrangement can range from about 90 degrees to about 190 degrees, for example. The inflation mechanism (not shown) selectably pressurizes and depressurizes the fluid within the inflation lumens 96 to respectively inflate and deflate the inflatable members 93.

It is noted that, with respect to the various embodiments described herein, a central lumen of the navigator catheter 94 can be used to receive an injection of a contrast media for mapping blood vessels. The navigator catheter 94 or guiding catheter 92, depending on the particular configuration, can thus be used to inject radiographic contrast media into the coronary sinus or other coronary vein to highlight the associated venous system.

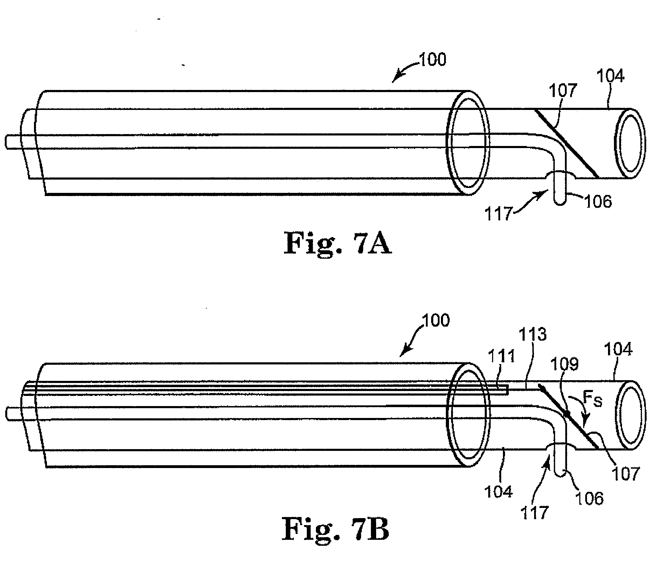

In accordance with another embodiment of the present invention, and with reference to Figs. 7A and 7B, a coronary vein guide catheter system 100 employs a navigator catheter 104 which includes a deflection member 107 situated proximate an aperture 117 of a wall of the navigator catheter 104. In general terms, the deflection member 107 is positioned within a central lumen of the navigator catheter 104 to contact a guide wire 106 being advanced through the navigator catheter 104. Upon contact, the deflection member 107 redirects the path of the guide wire 106 so that the guide wire 106 exits the aperture 117 at a desired exit angle appropriate for a coronary branch vein of interest.

As shown, the deflection member 107 of Fig. 7A is fixedly mounted at a prescribed angle so that the guide wire 106, upon contacting the deflection member 107, is directed through the aperture 117 at a prescribed exit angle. In the illustration of Fig. 7A, the deflection member 107 directs the guide wire 106 through the aperture 117 at an exit angle of about 90 degrees relative to a longitudinal axis of the navigation catheter 1.04. It is understood that acute or obtuse exit angles can be achieved by judicious selection of the orientation of the deflection member 107 within the central lumen of the navigation catheter 104.

Figure 7B illustrates a navigation catheter 104 employing an adjustable deflection member 107. In this configuration, a pull wire 113 disposed in a satellite lumen 111 is employed to control the deflection orientation of the deflection member 107. As shown, the deflection member 107 is pivotally mounted at a central axis 109 of the deflection member 107. A bias mechanism, such as a spring mechanism, is employed to produce a force, Fₛ, that opposes a proximally directed pull force on the pull wire 113. As such, the deflection member 107 provides for an initial deflection orientation when no pull force is applied to the pull wire 113. As shown, this initial deflection orientation results in a guide wire exit angle of about 90 degrees relative to a longitudinal axis of the navigation catheter 104. It is understood that the initial deflection orientation of the deflection member 107 can be selected to provide for an initial acute or obtuse exit angle.

Application of a pull force on the pull wire 113 causes the deflection member 107 to rotate about its pivot axis 109. As this pull force changes, the degree of deflection member rotation changes, thus providing for a concomitant change in the guide wire exit angle. It will be appreciated that a variety of guide wire exit angle ranges can be achieved by appropriate selection of deflection member size, positioning, initial deflection orientation, and range of rotation, among other considerations.

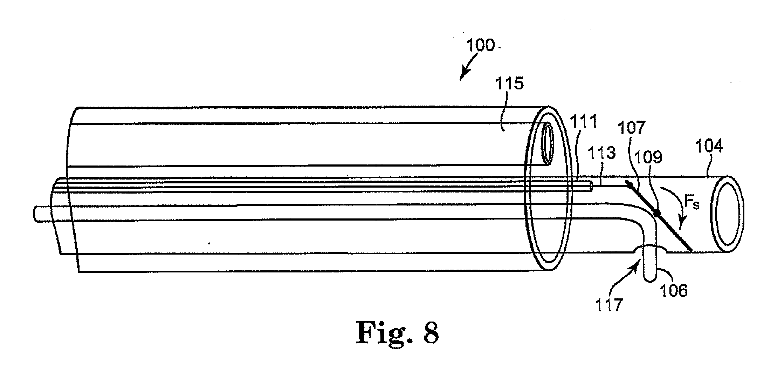

Figure 8 illustrates a coronary vein guide catheter system 100 that incorporates the features shown in Fig. 7B and further includes a satellite lumen 115. The satellite lumen 115 may be use for a variety of purposes, including accommodating a contrast media fluid, a sensor catheter or a shaping member, such as a stylet or shaping wire, for example.

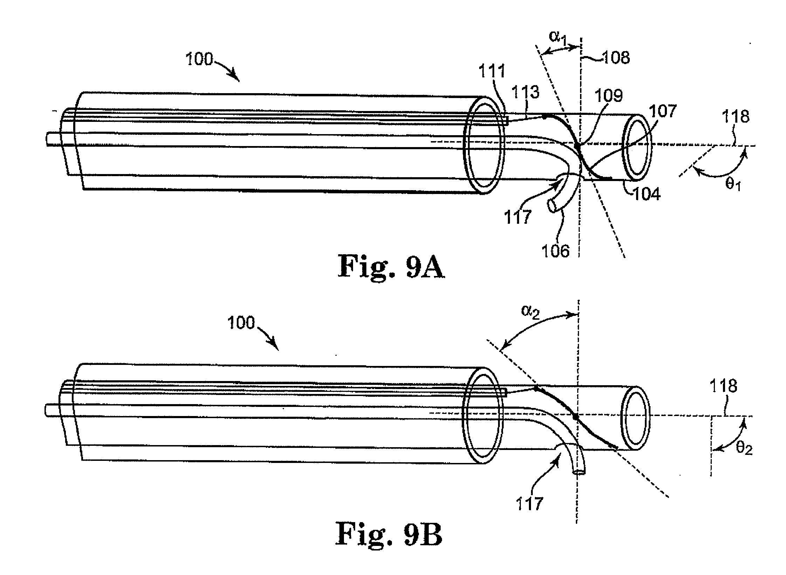

Figures 9A and 9B illustrate another configuration of a navigator catheter 104 that employs a controllable deflection member 107 similar to that described above with respect to Fig. 7B. According to this implementation, The deflection member 107 has a length greater than the diameter of the navigator catheter's central lumen, such that it takes on a S-shape when biased in its initial deflection orientation, as is shown in Fig. 9A. In this case, the deflection member 107 is orientated at an initial rotation angle, α₁, relative to vertical axis 108, which provides for a guide wire exit angle of θ₁ relative to horizontal axis 118.

When a pull force is applied to the pull wire 113, the deflection member 107 rotates, yet the opposing ends of the deflection member 107 advantageously maintain close contact with the guide catheter's inner walls. When fully rotated to orientation angle α₂, the deflection member 107 shown in Fig. 9B provides for a guide wire exit angle of θ₂ relative to horizontal axis 118. Continuous close contact between the deflection member 107 and walls of the navigator catheter's inner wall during deflection member movement improves the process of redirecting the path of the guide wire 106 into a sharply angled branch vein.

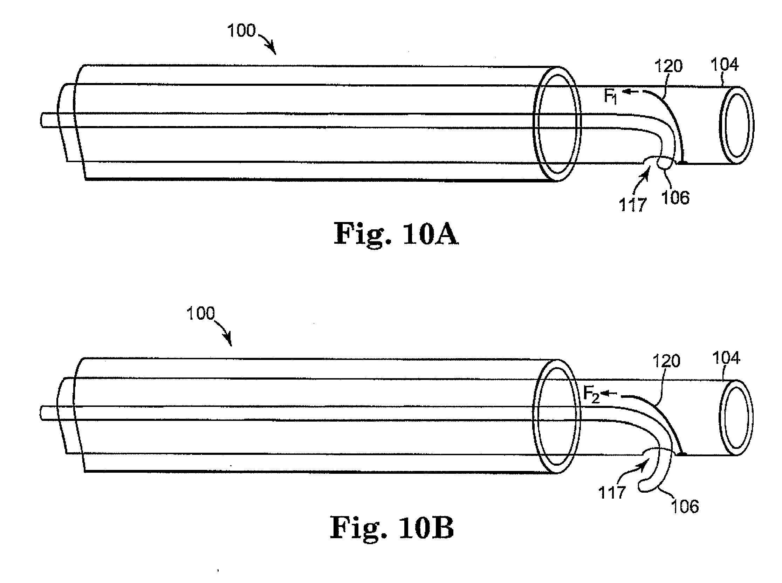

Figures 10A and 10B illustrate another implementation of a navigator catheter 104 that employs a deflection member 120 for redirecting a guide wire 106 at a desired exit angle through an exit aperture 117 of the catheter 104. According to this configuration, one end of the deflection member 120 is pivotally mounted at a mounting site on the inner wall of the navigator catheter's central lumen. The mounting site for the deflection member 120 is preferably immediately distal of the exit aperture 117. Application of a proximally directed force, such as forces F₁ or F₂, on the end of the deflection member 120 opposing the pivotally mounted end results in changing the deflection orientation of the deflection member 120, and thus the exit angle of the guide wire. The control forces F1 and F2 can be generated through use of pull wires or other known means.

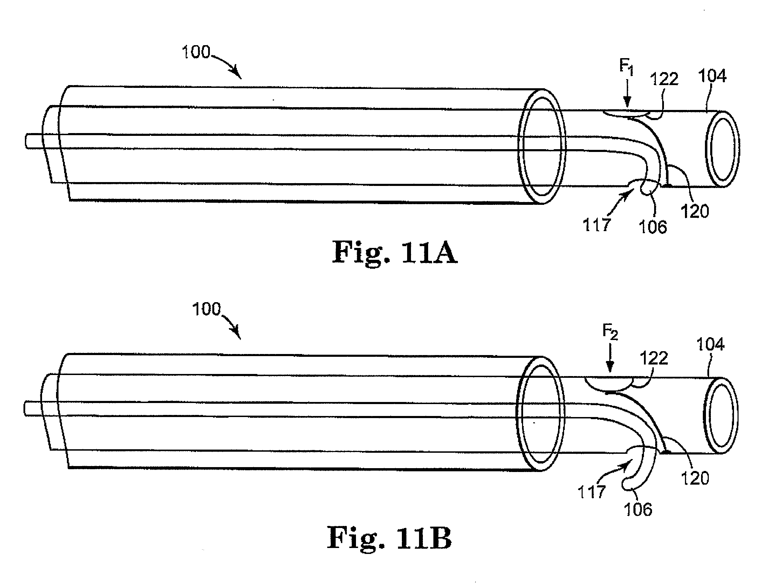

Figures 11A and 11 B illustrate yet another implementation of a navigator catheter 104 that employs a deflection member 120 for redirecting a guide wire 106 at a desired exit angle through an exit aperture 117 of the catheter 104. In this configuration, one end of the deflection member 120 is pivotally mounted at a mounting site on the inner wall of the navigator catheter's central lumen as discussed above. An inflation member 122 is situated on the inner wall of the navigator catheter's central lumen at a location opposing the exit aperture 117. The end of the deflection member 120 opposing the pivotally mounted end is in contact with the inflation member 122. The inflation member 122 can be selectably pressurized and depressurized to achieve a desired guide wire exit angle. One or more inflation lumens (not shown) and a proximal inflation mechanism (not shown) of the type previously described may be employed to controllably pressurize and depressurize the inflation member 122.

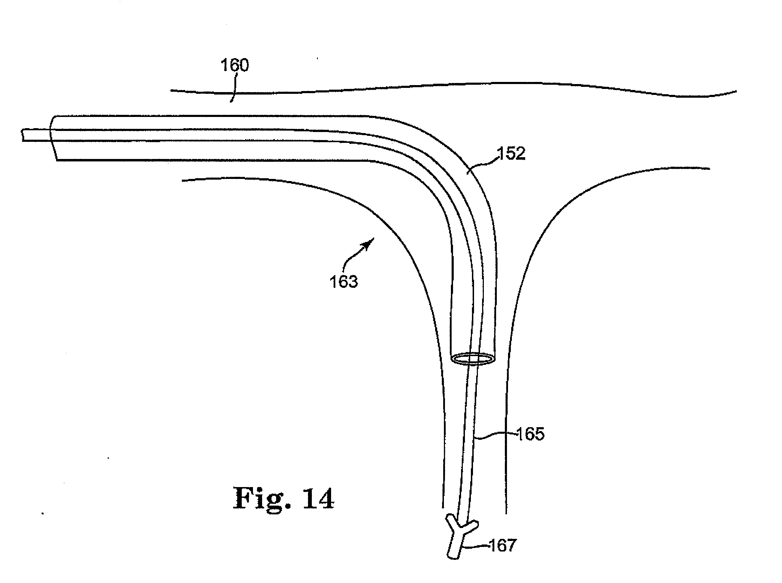

Figures 12-14 illustrate a further embodiment of the present invention. According to this embodiment, a coronary vein guide catheter system 150 includes a navigator catheter 154 movably extendable with respect to a guide catheter 152. The navigator catheter 154 shown in Figs. 12-14 can be fabricated to include many of the previously described features, as can the guiding catheter 152. For example, the guiding catheter 152 can include a pre-stress line 151 to facilitate peal-away retraction of the guide catheter 152 from the patient subsequent to lead implantation.

According to this embodiment, the navigator catheter 154 or navigator member (e.g., stylet) and guide catheter 152 are employed to access left-side coronary vasculature for implanting with or without use of a guide wire for over-the-wire lead implantation. The navigator catheter or member 152 is extended from the guide catheter 154, which is shown situated within the coronary sinus 160, to a position proximate a take off of a branch vein 162 distal to the coronary sinus ostium 160. The navigator member or catheter 154, which may have an open lumen or a closed lumen at its distal end, is maneuvered around the bend angle 163 of the branch vein 162 and advanced into the branch vein 162. In the case of an open lumen configuration, a relatively large diameter guide wire (not shown) can be advanced through the open lumen of the navigator catheter 154 to assist in accessing the branch vein 162. However, according to this embodiment, the guide wire is retracted after the navigator catheter 154 is advanced into the branch vein 162 and not used as part of the lead implant procedure.

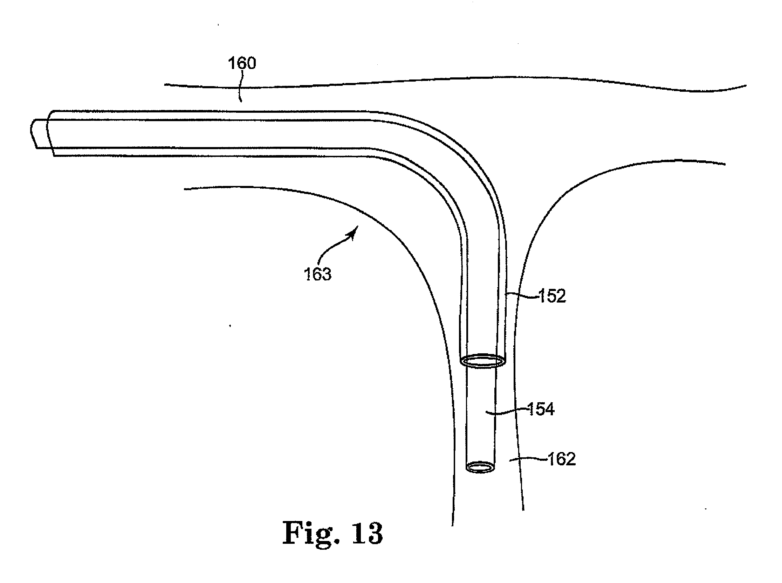

After the navigator catheter or member 154 is seated in the coronary branch vein 162, and as is best seen in Fig. 13, the guide catheter 152 is advanced over the navigator catheter or member 154 so that the guide catheter 152 is advanced past the bend angle 163 of the branch vein 162 and into the branch vein 162. The navigator catheter or member 164 is then retracted from the guide catheter 152, and a medical electrical lead 165 is advanced through the guide catheter 152. The lead electrode 167 is then implanted at the implant site, and the guide catheter 152 is removed.

It will, of course, be understood that various modifications and additions can be made to the preferred embodiments discussed hereinabove without departing from the scope of the present invention. Accordingly, the scope of the present invention should not be limited by the particular embodiments described above, but should be defined only by the claims set forth below and equivalents thereof.

This application is a divisional application of European patent application no. 03 793 298.2 (the "parent application"), also published under no. WO 2004/018029 A2. The original claims of the parent application are repeated below in the present specification and form part of the content of this divisional application as filed.
1. A guide catheter system, comprising:
   a guide catheter comprising a flexible shaft defining a longitudinal axis and having a proximal end, a distal end, and a main lumen;
   a navigator catheter having a proximal end, a distal end, and a central lumen, the navigator catheter longitudinally displaceable within the main lumen of the guide catheter, the distal end of the navigator catheter dimensioned for passage into an angled vein distal to a patient's coronary sinus ostium, the central lumen dimensioned to receive a longitudinally displaceable guide wire; and
   a deflection arrangement provided at the distal end of the navigator catheter for directing the guide wire into the angled vein, the deflection arrangement imparting a bend at the distal end of the navigator catheter having an angle sufficient to facilitate passage of the distal end of the navigator catheter into the angled vein.
2. The system of claim 1, wherein the bend angle is an acute angle relative to a longitudinal axis of the navigator catheter proximal of the deflection arrangement.
3. The system of claim 1, wherein the bend angle is an obtuse angle relative to a longitudinal axis of the navigator catheter proximal of the deflection arrangement.
4. The system of claim 1, wherein the bend angle ranges between about 0 degrees and about 180 degrees relative to a longitudinal axis of the navigator catheter proximal of the deflection arrangement.
5. The system of claim 1, wherein the deflection arrangement comprises a pre-formed region of the distal end of the navigator catheter, the pre-formed region assuming the bend angle when the pre-formed region extends beyond the distal end of the guide catheter.
6. The system of claim 5, wherein the pre-formed distal end of the navigator catheter is more flexible than the distal end of the guide catheter.
7. The system of claim 1, wherein the distal end of the guide catheter comprises a pre-formed region.
8. The system of claim 1, wherein the deflection arrangement comprises a deflection mechanism that imparts the bend angle, the deflection mechanism controllable from the proximal end of the navigator catheter.
9. The system of claim 8, wherein the deflection mechanism comprises a deflection tendon attached at or proximate a distal tip of the navigator catheter and extending to the proximal end of the navigator catheter.
10. The system of claim 9, wherein a plurality of bend angles are developed upon application of forces to the deflection tendon.
11. The system of claim 1, wherein the deflection arrangement comprises a flexible region at the distal end of the navigator catheter and a shaping member longitudinally displaceable within the central lumen of the navigator catheter, the flexible region assuming the bend angle as the shaping member passes into the flexible region.
12. The system of claim 11, wherein the shaping member comprises a shaping wire.
13. The system of claim 11, wherein the shaping member comprises a stylet.
14. The system of claim 1, wherein the deflection arrangement comprises an inflation member encompassing at least part of the distal end of the navigator catheter, an inflation lumen of the navigator catheter fluidly coupling the inflation member with an inflation mechanism provided at the proximal end of the navigator catheter, the inflation mechanism selectably pressurizing and depressurizing a fluid within the inflation lumen to respectively inflate and deflate the inflatable member to develop a desired bend angle.
15. The system of claim 1, wherein the guide catheter has an outer diameter of about 10 French or less, and the navigator catheter has an outer diameter of about 8 French or less.
16. The system of claim 1, wherein the guide catheter comprises a longitudinal pre-stress line extending between the distal and proximal ends of the guide catheter, the guide catheter splitting along the longitudinal pre-stress line upon guide catheter retraction in a proximal direction.
17. The system of claim 1, wherein the central lumen of the navigation catheter is configured to receive a contrast media for mapping vasculature.
18. A guide catheter system, comprising:
   a guide catheter comprising a flexible shaft defining a longitudinal axis and having a proximal end, a distal end, and a main lumen;
   a navigator member having a proximal end and a distal end, the navigator member longitudinally displaceable within the main lumen of the guide catheter, the distal end of the navigator member dimensioned for passage into an angled vein distal to a patient's coronary sinus ostium; and
   a deflection arrangement provided at the distal end of the navigator member, the deflection arrangement imparting a bend at the distal end of the navigator member having an angle sufficient to facilitate passage of the distal end of the navigator member into the angled vein.
19. The system of claim 18, wherein the bend angle is an acute angle relative to a longitudinal axis of the navigator catheter proximal of the deflection arrangement.
20. The system of claim 18, wherein the bend angle is an obtuse angle relative to a longitudinal axis of the navigator catheter proximal of the deflection arrangement.
21. The system of claim 18, wherein the bend angle ranges between about 0 degrees to about 180 degrees relative to a longitudinal axis of the navigator catheter proximal of the deflection arrangement.
22. The system of claim 18, wherein the deflection arrangement comprises a pre-formed region of the distal end of the navigator member, the pre-formed region assuming the bend angle when the pre-formed region extends beyond the distal end of the guide member.
23. The system of claim 18, wherein the distal end of the guide catheter comprises a pre-formed region.
24. The system of claim 18, wherein the navigator member comprises a navigator catheter and the deflection arrangement comprises a deflection mechanism that imparts the bend angle, the deflection mechanism controllable from the proximal end of the navigator member.
25. The system of claim 24, wherein the deflection mechanism comprises a deflection tendon attached at or proximate a distal tip of the navigator catheter and extending to the proximal end of the navigator catheter.
26. The system of claim 25, wherein a plurality of bend angles are developed upon application of forces to the deflection tendon.
27. The system of claim 18, wherein the navigator member comprises a navigator catheter, and the deflection arrangement comprises a flexible region at the distal end of the navigator catheter and a shaping member longitudinally displaceable within a central lumen of the navigator catheter, the flexible region assuming the bend angle as the shaping member passes into the flexible region.
28. The system of claim 27, wherein the shaping member comprises a shaping wire.
29. The system of claim 27, wherein the shaping member comprises a stylet.
30. The system of claim 18, wherein the navigator member comprises a navigator catheter and the deflection arrangement comprises an inflation member encompassing at least part of the distal end of the navigator catheter, an inflation lumen of the navigator catheter fluidly coupling the inflation member with an inflation mechanism provided at the proximal end of the navigator catheter, the inflation mechanism selectably pressurizing and depressurizing a fluid within the inflation lumen to respectively inflate and deflate the inflatable member to develop a desired bend angle.
31. The system of claim 18, wherein the guide catheter has an outer diameter of about 10 French or less, and the navigator member has an outer diameter of about 8 French or less.
32. The system of claim 18, wherein the guide catheter comprises a longitudinal pre-stress line extending between the distal and proximal ends of the guide catheter, the guide catheter splitting along the longitudinal pre-stress line upon guide catheter retraction in a proximal direction.
33. The system of claim 18, wherein the navigator member comprises a navigator catheter, the navigation catheter further comprising a lumen through which a contrast media can be communicated for mapping vasculature.
34. A guide catheter system, comprising:
   a guide catheter comprising a flexible shaft and having a proximal end, a distal end, and a main lumen;
   a navigator catheter having an outer wall including an aperture, a central lumen, a proximal end, and a distal end, the navigator catheter longitudinally displaceable within the main lumen of the guide catheter, the distal end of the navigator catheter dimensioned for passage into a cardiac vein distal to a patient's coronary sinus ostium; and
   a deflection member disposed within the central lumen of the navigator catheter proximate the aperture of the outer wall, the deflection member oriented at an angle relative to a longitudinal axis of the navigator catheter sufficient to deflect a guide wire passed within the central lumen through the aperture of the outer wall of the navigator catheter and into an angled vein branching from the cardiac vein.
35. The system of claim 34, wherein the angle of the deflection member is an acute angle relative to the longitudinal axis of the navigation catheter.
36. The system of claim 34, wherein the angle of the deflection member is an obtuse angle relative to the longitudinal axis of the navigation catheter.
37. The system of claim 34, wherein the deflection member is fixedly mounted within the central lumen.
38. The system of claim 34, wherein the deflection member is movably mounted to assume a plurality of angels relative to the longitudinal axis of the navigator catheter sufficient to deflect the guide wire through the aperture of the outer wall of the navigator catheter at a plurality of exit angles.
39. The system of claim 34, wherein the deflection member is pivotably mounted within the central lumen to assume a plurality of angels relative to the longitudinal axis of the navigator catheter sufficient to deflect the guide wire through the aperture of the outer wall of the navigator catheter at a plurality of exit angles.
40. The system of claim 39, wherein a central axis of the deflection member defines a pivot axis of the deflection member.
41. The system of claim 39, wherein the deflection member comprises a pivot axis, the pivot axis defined by a location at which one end of the deflection member is pivotally connected to an inner wall of the central lumen.
42. The system of claim 39, wherein the deflection member is connected to a deflection tendon, and application of forces to the deflection tendon causes the deflection member to pivot about a pivot axis.
43. The system of claim 42, wherein the deflection member comprises a bias mechanism, the bias mechanism generating a force opposing those applied to the deflection tendon.
44. The system of claim 34, wherein the guide catheter has an outer diameter of about 8 French or less.
45. The system of claim 34, wherein the guide catheter comprises a longitudinal pre-stress line extending between the distal and proximal ends of the guide catheter, the guide catheter splitting along the longitudinal pre-stress line upon guide catheter retraction in a proximal direction.
46. The system of claim 34, wherein the guide catheter further comprises a lumen through which a contrast media can be communicated for mapping vasculature.
47. A method of navigating coronary vasculature, comprising:
   providing a guide catheter system comprising a guide catheter, a navigator catheter longitudinally displaceable within the guide catheter, and a deflection arrangement provided at a distal end of the navigator catheter;
   advancing the guide catheter to at least a patient's coronary sinus ostium;
   extending the navigator catheter from the guide catheter to a location proximate or within an angled vein distal to the coronary sinus ostium;
   using the deflection arrangement to direct a guide wire passing through the navigation catheter into the angled vein; and
   advancing a lead having an open lumen over the guide wire to direct the lead to an implant site within the angled vein.
48. The method of claim 47, wherein using the deflection arrangement comprises using a pre-shaped distal bend at a distal end of the navigator catheter to direct the guide wire into the angled vein.
49. The method of claim 48, wherein the pre-shaped distal bend directs the guide wire into the angled vein at an acute angle relative to a longitudinal axis of the navigator catheter proximal of the pre-shaped distal bend.
50. The method of claim 48, wherein the pre-shaped distal bend directs the guide wire into the angled vein at a obtuse angle relative to a longitudinal axis of the navigator catheter proximal of the pre-shaped distal bend.
51. The method of claim 47, wherein using the deflection arrangement comprises using a shaping member to impart a pre-determined shape on a flexible distal end of the navigation catheter.
52. The method of claim 47, wherein using the deflection arrangement comprises changing a bend angle at a distal end region of the navigator catheter.
53. The method of claim 47, wherein using the deflection arrangement comprises changing a shape of a distal end region of the navigator catheter.
54. The method of claim 47, wherein using the deflection arrangement comprises controlling the deflection arrangement to direct the guide wire into the angled vein using a physician controlled deflection angle.
55. The method of claim 54, wherein controlling the deflection arrangement comprises controllably pressurizing and depressurizing the deflection arrangement to control the deflection angle.
56. The method of claim 54, wherein controlling the deflection arrangement comprises controllably changing an orientation of the deflection arrangement to control the deflection angle.
57. The method of claim 54, wherein controlling the deflection arrangement comprises controllably pivoting the deflection arrangement to control the deflection angle.
58. The method of claim 47, further comprising communicating a contrast dye through the navigator catheter to facilitate blood vessel mapping.
59. The method of claim 47, further comprising splitting the guide catheter at a proximal end while retracting the guide catheter from the patient.
60. A method of navigating coronary vasculature, comprising:
   providing a guide catheter system comprising a guide catheter, a navigator catheter longitudinally displaceable within the guide catheter, and a deflection arrangement provided at a distal end of the navigator catheter;
   advancing the guide catheter to at least a patient's coronary sinus ostium;
   extending the navigator catheter from the guide catheter to a location proximate an angled vein distal to the coronary sinus ostium;
   seating the navigator catheter within the angled vein;
   passing the guide catheter over the navigator catheter to advance the guide catheter into the angled vein;
   retracting the navigator catheter from the guide catheter, and
   advancing a lead through the guide catheter to an implant site within the angled vein.
61. The method of claim 60, further comprising using a guide wire passing through and beyond the navigator catheter to assist in locating one or both of the coronary sinus ostium and the angled vein.
62. The method of claim 60, wherein seating the navigator catheter comprises using a pre-shaped distal bend at a distal end of the navigator catheter to access the angled vein.
63. The method of claim 62, wherein the pre-shaped distal bend defines an acute angle relative to a longitudinal axis of the navigator catheter proximal of the pre-shaped distal bend.
64. The method of claim 62, wherein the pre-shaped distal bend defines an obtuse angle relative to a longitudinal axis of the navigator catheter proximal of the pre-shaped distal bend.
65. The method of claim 60, wherein seating the navigator catheter comprises using a shaping member to impart a pre-determined shape on a flexible distal end of the navigation catheter, and using the shaped flexible distal end of the navigator catheter to access the angled vein.
66. The method of claim 60, wherein seating the navigator catheter comprises changing a bend angle at a distal end region of the navigator catheter.
67. The method of claim 60, wherein seating the navigator catheter comprises changing a shape of a distal end region of the navigator catheter.
68. The method of claim 60, wherein seating the navigator catheter comprises controlling a deflection arrangement proximate a distal end of the navigator catheter to access the angled vein.
69. The method of claim 68, wherein controlling the deflection arrangement comprises controllably pressurizing and depressurizing the deflection arrangement to control a deflection angle of a distal portion of the navigator catheter.
70. The method of claim 60, further comprising communicating a contrast dye through the navigator catheter to facilitate blood vessel mapping.
71. The method of claim 60, further comprising splitting the guide catheter at a proximal end while retracting the guide catheter from the patient.

## Claims

1. A guide catheter system, comprising:
a guide catheter comprising a flexible shaft defining a longitudinal axis and having a proximal end, a distal end, and a main lumen;
a navigator catheter having a proximal end, a distal end, and a central lumen, the navigator catheter longitudinally displaceable within the main lumen of the guide catheter, the distal end of the navigator catheter dimensioned for passage into an angled vein distal to a patient's coronary sinus ostium, the central lumen dimensioned to receive a longitudinally displaceable guide wire; and
a deflection arrangement provided at the distal end of the navigator catheter for directing the guide wire into the angled vein, the deflection arrangement imparting a bend at the distal end of the navigator catheter having an angle sufficient to facilitate passage of the distal end of the navigator catheter into the angled vein,
wherein the deflection arrangement comprises a deflection mechanism that imparts the bend angle, the deflection mechanism controllable from the proximal end of the navigator catheter.

2. The system of claim 1, wherein the deflection mechanism comprises a deflection tendon attached at or proximate a distal tip of the navigator catheter and extending to the proximal end of the navigator catheter.

3. The system of claim 2, wherein a plurality of bend angles are developed upon application of forces to the deflection tendon.

4. A guide catheter system, comprising:
a guide catheter comprising a flexible shaft defining a longitudinal axis and having a proximal end, a distal end, and a main lumen;
a navigator catheter having a proximal end, a distal end, and a central lumen, the navigator catheter longitudinally displaceable within the main lumen of the guide catheter, the distal end of the navigator catheter dimensioned for passage into an angled vein distal to a patient's coronary sinus ostium, the central lumen dimensioned to receive a longitudinally displaceable guide wire; and
a deflection arrangement provided at the distal end of the navigator catheter for directing the guide wire into the angled vein, the deflection arrangement imparting a bend at the distal end of the navigator catheter having an angle sufficient to facilitate passage of the distal end of the navigator catheter into the angled vein, wherein the deflection arrangement comprises a flexible region at the distal end of the navigator catheter and a shaping member longitudinally displaceable within the central lumen of the navigator catheter, the flexible region assuming the bend angle as the shaping member passes into the flexible region.

5. The system of claim 4, wherein the shaping member comprises a shaping wire.

6. The system of claim 4, wherein the shaping member comprises a stylet.

7. A guide catheter system, comprising:
a guide catheter comprising a flexible shaft defining a longitudinal axis and having a proximal end, a distal end, and a main lumen;
a navigator catheter having a proximal end, a distal end, and a central lumen, the navigator catheter longitudinally displaceable within the main lumen of the guide catheter, the distal end of the navigator catheter dimensioned for passage into an angled vein distal to a patient's coronary sinus ostium, the central lumen dimensioned to receive a longitudinally displaceable guide wire; and
a deflection arrangement provided at the distal end of the navigator catheter for directing the guide wire into the angled vein, the deflection arrangement imparting a bend at the distal end of the navigator catheter having an angle sufficient to facilitate passage of the distal end of the navigator catheter into the angled vein, wherein the deflection arrangement comprises an inflation member encompassing at least part of the distal end of the navigator catheter, an inflation lumen of the navigator catheter fluidly coupling the inflation member with an inflation mechanism provided at the proximal end of the navigator catheter, the inflation mechanism selectably pressurizing and depressurizing a fluid within the inflation lumen to respectively inflate and deflate the inflatable member to develop a desired bend angle.

8. A guide catheter system, comprising:
a guide catheter comprising a flexible shaft defining a longitudinal axis and having a proximal end, a distal end, and a main lumen;
a navigator member having a proximal end and a distal end, the navigator member longitudinally displaceable within the main lumen of the guide catheter, the distal end of the navigator member dimensioned for passage into an angled vein distal to a patient's coronary sinus ostium; and
a deflection arrangement provided at the distal end of the navigator member, the deflection arrangement imparting a bend at the distal end of the navigator member having an angle sufficient to facilitate passage of the distal end of the navigator member into the angled vein,
wherein the navigator member comprises a navigator catheter and the deflection arrangement comprises a deflection mechanism that imparts the bend angle, the deflection mechanism controllable from the proximal end of the navigator member.

9. The system of claim 8, wherein the deflection mechanism comprises a deflection tendon attached at or proximate a distal tip of the navigator catheter and extending to the proximal end of the navigator catheter.

10. The system of claim 9, wherein a plurality of bend angles are developed upon application of forces to the deflection tendon.

11. A guide catheter system, comprising:
a guide catheter comprising a flexible shaft defining a longitudinal axis and having a proximal end, a distal end, and a main lumen ;
a navigator member having a proximal end and a distal end, the navigator member longitudinally displaceable within the main lumen of the guide catheter, the distal end of the navigator member dimensioned for passage into an angled vein distal to a patient's coronary sinus ostium; and
a deflection arrangement provided at the distal end of the navigator member, the deflection arrangement imparting a bend at the distal end of the navigator member having an angle sufficient to facilitate passage of the distal end of the navigator member into the angled vein, wherein the navigator member comprises a navigator catheter, and the deflection arrangement comprises a flexible region at the distal end of the navigator catheter and a shaping member longitudinally displaceable within a central lumen of the navigator catheter, the flexible region assuming the bend angle as the shaping member passes into the flexible region.

12. The system of claim 11, wherein the shaping member comprises a shaping wire.

13. The system of claim 11, wherein the shaping member comprises a stylet.

14. A guide catheter system, comprising:
a guide catheter comprising a flexible shaft defining a longitudinal axis and having a proximal end, a distal end, and a main lumen ;
a navigator member having a proximal end and a distal end, the navigator member longitudinally displaceable within the main lumen of the guide catheter, the distal end of the navigator member dimensioned for passage into an angled vein distal to a patient's coronary sinus ostium; and
a deflection arrangement provided at the distal end of the navigator member, the deflection arrangement imparting a bend at the distal end of the navigator member having an angle sufficient to facilitate passage of the distal end of the navigator member into the angled vein, wherein the navigator member comprises a navigator catheter and the deflection arrangement comprises an inflation member encompassing at least part of the distal end of the navigator catheter, an inflation lumen of the navigator catheter fluidly coupling the inflation member with an inflation mechanism provided at the proximal end of the navigator catheter, the inflation mechanism selectably pressurizing and depressurizing a fluid within the inflation lumen to respectively inflate and deflate the inflatable member to develop a desired bend angle.

15. A guide catheter system, comprising: a guide catheter comprising a flexible shaft and having a proximal end, a distal end, and a main lumen ; a navigator catheter having an outer wall including an aperture, a central lumen, a proximal end, and a distal end, the navigator catheter longitudinally displaceable within the main lumen of the guide catheter, the distal end of the navigator catheter dimensioned for passage into a cardiac vein distal to a patient's coronary sinus ostium; and a deflection member disposed within the central lumen of the navigator catheter proximate the aperture of the outer wall, the deflection member oriented at an angle relative to a longitudinal axis of the navigator catheter sufficient to deflect a guide wire passed within the central lumen through the aperture of the outer wall of the navigator catheter and into an angled vein branching from the cardiac vein.

16. The system of claim 15, wherein the angle of the deflection member is an acute angle relative to the longitudinal axis of the navigation catheter.

17. The system of claim 15, wherein the angle of the deflection member is an obtuse angle relative to the longitudinal axis of the navigation catheter.

18. The system of claim 15, wherein the deflection member is fixedly mounted within the central lumen.

19. The system of claim 15, wherein the deflection member is movably mounted to assume a plurality of angels relative to the longitudinal axis of the navigator catheter sufficient to deflect the guide wire through the aperture of the outer wall of the navigator catheter at a plurality of exit angles.

20. The system of claim 15, wherein the deflection member is pivotably mounted within the central lumen to assume a plurality of angels relative to the longitudinal axis of the navigator catheter sufficient to deflect the guide wire through the aperture of the outer wall of the navigator catheter at a plurality of exit angles.

21. The system of claim 20, wherein a central axis of the deflection member defines a pivot axis of the deflection member.

22. The system of claim 20, wherein the deflection member comprises a pivot axis, the pivot axis defined by a location at which one end of the deflection member is pivotally connected to an inner wall of the central lumen.

23. The system of claim 20, wherein the deflection member is connected to a deflection tendon, and application of forces to the deflection tendon causes the deflection member to pivot about a pivot axis.

24. The system of claim 23, wherein the deflection member comprises a bias mechanism, the bias mechanism generating a force opposing those applied to the deflection tendon.

25. The system of claim 15, wherein the guide catheter has an outer diameter of about 8 French or less.

26. The system of claim 15, wherein the guide catheter comprises a longitudinal pre-stress line extending between the distal and proximal ends of the guide catheter, the guide catheter splitting along the longitudinal pre-stress line upon guide catheter retraction in a proximal direction.

27. The system of claim 15, wherein the guide catheter further comprises a lumen through which a contrast media can be communicated for mapping vasculature.
